(19) Europäisches Patentamt
European Patent Office
Office européen des brevets

(11) Veröffentlichungsnummer: **0 443 162 A1**

(12) **EUROPÄISCHE PATENTANMELDUNG**

(21) Anmeldenummer: **90124682.7**

(22) Anmeldetag: **19.12.90**

(51) Int. Cl.5: **C07D 231/08**, C07D 231/54, A01N 43/56

(30) Priorität: **17.02.90 DE 4005114**

(43) Veröffentlichungstag der Anmeldung: **28.08.91 Patentblatt 91/35**

(84) Benannte Vertragsstaaten: **BE CH DE FR GB IT LI NL**

(71) Anmelder: **BAYER AG**

**W-5090 Leverkusen 1 Bayerwerk(DE)**

(72) Erfinder: **Fuchs, Rainer, Dr.**
**Am Rohm 107**
**W-5600 Wuppertal 1(DE)**
Erfinder: **Erdelen, Christoph, Dr.**
**Unterbüscherhof 22**
**W-5653 Leichlingen 3(DE)**
Erfinder: **Becker, Benedikt, Dr.**
**Via Max Sparer 17**
**I-39057 Appiano (BZ)(IT)**
Erfinder: **Stendel, Wilhelm, Dr.**
**In den Birken 55**
**W-5600 Wuppertal 1(DE)**

(54) **Substituierte Pyrazolinderivate.**

(57) Es werden neue substituierte Pyrazolinderivate der allgemeinen Formel

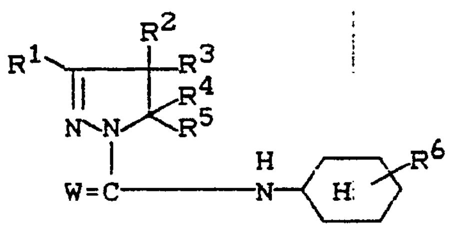

(I)

bereitgestellt,
in welcher

$R^1$ für gegebenenfalls substituiertes Phenyl oder gegebenenfalls substituiertes Alkyl steht,
$R^2$ für Wasserstoff, gegebenenfalls substituiertes Phenyl, gegebenenfalls substituiertes Alkyl oder Alkoxycarbonyl steht,
oder $R^1$ und $R^2$ zusammen für einen gegebenenfalls benzoanellierten Alkylenrest stehen,
$R^3$ für Wasserstoff oder Alkyl steht,
$R^4$ für Wasserstoff, gegebenenfalls substituiertes Phenyl oder gegebenenfalls substituiertes Alkyl steht,
$R^5$ für Wasserstoff oder gegebenenfalls substituiertes Alkyl steht,
$R^6$ für Halogenalkyl oder Halogenalkoxy steht und
W für Sauerstoff oder Schwefel steht.

Die neuen Verbindungen besitzen eine stark ausgeprägte Wirksamkeit gegen tierische Schädlinge, insbesondere gegen Insekten und Spinnentiere.

EP 0 443 162 A1

## SUBSTITUIERTE PYRAZOLINDERIVATE

Die Erfindung betrifft neue substituierte Pyrazolinderivate, ein Verfahren zu ihrer Herstellung und ihre Verwendung als Schädlingsbekämpfungsmittel.

Es ist bekannt, daß bestimmte substituierte Pyrazolinderivate eine gute Wirksamkeit gegen tierische Schädlinge besitzen.

Siehe dazu z.B. DE-A 2 700 258, US-A 4 174 393, DE-A 2 529 689, US-A 4 070 365.

Die Wirkungshöhe bzw. Wirkungsdauer dieser vorbekannten Verbindungen ist jedoch, insbesondere gegen bestimmte Organismen oder bei niedrigen Anwendungskonzentrationen, nicht in allen Anwendungsgebieten völlig zufriedenstellend.

Es wurden neue substituierte Pyrazolinderivate der allgemeinen Formel (I)

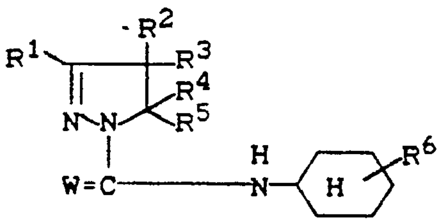

(I)

gefunden, in welcher

- $R^1$ für gegebenenfalls substituiertes Phenyl oder gegebenenfalls substituiertes Alkyl steht,
- $R^2$ für Wasserstoff, gegebenenfalls substituiertes Phenyl, gegebenenfalls substituiertes Alkyl oder Alkoxycarbonyl steht,

oder $R^1$ und $R^2$ zusammen für einen gegebenenfalls benzoanellierten Alkylenrest stehen,

- $R^3$ für Wasserstoff oder Alkyl steht,
- $R^4$ für Wasserstoff, gegebenenfalls substituiertes Phenyl oder gegebenenfalls substituiertes Alkyl steht,
- $R^5$ für Wasserstoff oder gegebenenfalls substituiertes Alkyl steht,
- $R^6$ für Halogenalkyl oder Halogenalkoxy steht und
- W für Sauerstoff oder Schwefel steht.

Weiterhin wurde gefunden, daß man die neuen substituierten Pyrazolinderivate der allgemeinen Formel (I)

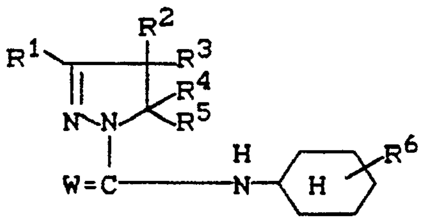

(I)

in welcher

- $R^1$ für gegebenenfalls substituiertes Phenyl oder gegebenenfalls substituiertes Alkyl steht,
- $R^2$ für Wasserstoff, gegebenenfalls substituiertes Phenyl, gegebenenfalls substituiertes Alkyl oder Alkoxycarbonyl steht,

oder $R^1$ und $R^2$ zusammen für einen gegebenenfalls benzoanellierten Alkylenrest stehen,

- $R^3$ für Wasserstoff oder Alkyl steht,
- $R^4$ für Wasserstoff, gegebenenfalls substituiertes Phenyl oder gegebenenfalls substituiertes Alkyl steht,
- $R^5$ für Wasserstoff oder gegebenenfalls substituiertes Alkyl steht,
- $R^6$ für Halogenalkyl oder Halogenalkoxy steht und
- W für Sauerstoff oder Schwefel steht,

erhält, wenn man Pyrazolinderivate der Formel (II)

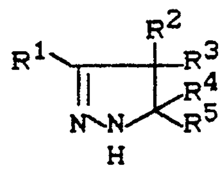

(II)

in welcher
$R^1$, $R^2$, $R^3$, $R^4$ und $R^5$ die oben angegebene Bedeutung besitzen,
mit Isocyanaten bzw. Isothiocyanaten der Formel (III)

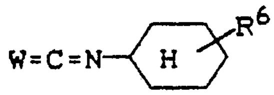

(III)

in welcher
W und $R^6$ die oben angegebene Bedeutung haben,
gegebenenfalls in Gegenwart von Basen umsetzt.

Schließlich wurde gefunden, daß die neuen Pyrazolinderivate der allgemeinen Formel (I) eine sehr gute Wirksamkeit gegen Schädlinge und insbesondere eine sehr gute insektizide und akarizide Wirksamkeit besitzen.

Überraschenderweise zeigen die erfindungsgemäßen substituierten Pyrazolinderivate eine erheblich bessere insektizide Wirksamkeit gegen pflanzenschädigende und warmblüterparasitierende Insekten und Spinnentiere als aus dem Stand der Technik bekannte chemisch und wirkungsmäßig naheliegende Verbindungen.

Die erfindungsgemäßen substituierten Pyrazolinderivate sind durch die Formel (I) allgemein definiert.
Bevorzugt sind Verbindungen der Formel (I), bei welchen

- $R^1$ für einen gegebenenfalls durch Halogen, Nitro, Alkyl($C_1$-$C_6$), Alkoxy($C_1$-$C_6$), Halogenalkyl($C_1$-$C_6$), Halogenalkoxy($C_1$-$C_6$), Alkyl($C_1$-$C_6$)sulfonyl, Halogenalkyl($C_1$ -$C_6$)sulfonyl substituiertes Phenyl oder für gegebenenfalls durch Halogen, Alkoxy($C_1$-$C_6$), Halogenalkoxy($C_1$-$C_6$) substituiertes Alkyl-($C_1$-$C_6$) steht,
- $R^2$ für Wasserstoff, gegebenenfalls durch Halogen, Nitro, Alkyl($C_1$-$C_6$), Alkoxy($C_1$-$C_6$), Halogenalkyl-($C_1$ -$C_6$), Halogenalkoxy($C_1$-$C_6$), Alkyl($C_1$-$C_6$)sulfonyl, Halogenalkyl($C_1$-$C_6$)sulfonyl substituiertes Phenyl, für gegebenenfalls durch Halogen, Alkoxy($C_1$ -$C_6$), Halogenalkoxy($C_1$-$C_6$) substituiertes Alkyl-($C_1$ -$C_6$) oder für Alkoxy($C_1$-$C_6$)carbonyl steht,

oder wobei

- $R^3$ für Wasserstoff oder Alkyl($C_1$-$C_6$) steht,
- $R^4$ für Wasserstoff, gegebenenfalls durch Halogen, Alkoxy($C_1$-$C_4$), Alkyl($C_1$-$C_4$), Halogenalkyl($C_1$-$C_4$), Halogenalkoxy($C_1$-$C_4$) substituiertes Phenyl oder für gegebenenfalls durch Halogen, Alkoxy($C_1$-$C_4$) substituiertes Alkyl($C_1$-$C_6$) steht,
- $R^5$ für Wasserstoff oder gegebenenfalls durch Halogen, Alkoxy($C_1$-$C_4$) substituiertes Alkyl($C_1$-$C_6$) steht,
- $R^6$ für Halogenalkyl($C_1$-$C_6$) oder Halogenalkoxy($C_1$-$C_6$) steht

und

- W für Sauerstoff oder Schwefel steht.

Besonders bevorzugt sind Verbindungen der Formel (I), in welcher

- $R^1$ für gegebenenfalls durch Fluor, Chlor, Brom, Nitro, Alkyl($C_1$-$C_4$), Alkoxy($C_1$-$C_4$), Halogenalkyl($C_1$-$C_4$), Halogenalkoxy($C_1$-$C_4$), Alkyl($C_1$-$C_4$)sulfonyl, Halogenalkyl($C_1$ -$C_4$)sulfonyl, Halogenalkoxy($C_1$-$C_4$)sulfonyl substituiertes Phenyl oder für gegebenenfalls durch Fluor, Chlor, Brom, Alkoxy($C_1$-$C_4$), Halogenalkoxy($C_1$ -$C_4$) substituiertes Alkyl($C_1$-$C_4$) steht,
- $R^2$ für Wasserstoff, gegebenenfalls durch Fluor, Chlor, Brom, Nitro, Alkyl($C_1$-$C_4$), Alkoxy($C_1$-$C_4$), Halogenalkyl($C_1$ -$C_4$), Halogenalkoxy($C_1$-$C_4$), Alkyl($C_1$-$C_4$)-sulfonyl, Halogenalkyl($C_1$-$C_4$)sulfonyl substituiertes Phenyl steht, für gegebenenfalls durch Fluor, Chlor, Brom, Alkoxy($C_1$-$C_4$), Halogenalkoxy($C_1$-$C_4$) substituiertes Alkyl($C_1$-$C_4$) oder für Alkoxy-($C_1$ -$C_4$)carbonyl steht,
- $R^3$ für Wasserstoff oder Alkyl($C_1$-$C_4$) steht,
- $R^4$ für Wasserstoff, gegebenenfalls durch Fluor, Chlor, Brom, Methoxy, Ethoxy, Methyl, Ethyl, Halogenalkyl($C_1$ -$C_2$), Halogenalkoxy($C_1$-$C_2$) substituiertes Phenyl oder für gegebenenfalls durch

Fluor, Chlor, Brom, Methoxy, Ethoxy substituiertes Alkyl($C_1$-$C_4$) steht,

$R^5$ für Wasserstoff oder gegebenenfalls durch Fluor, Chlor, Brom, Methoxy, Ethoxy substituiertes Alkyl-($C_1$ -$C_4$) steht,

$R^6$ für Halogenalkyl mit 1 bis 4 Kohlenstoffatomen und 1 bis 7 gleichen oder verschiedenen Halogenatomen der Reihe Fluor, Chlor, Brom oder für Halogenalkoxy mit 1 bis 4 Kohlenstoffatomen und 1 bis 7 gleichen oder verschiedenen Halogenatomen der Reihe Fluor, Chlor, Brom steht und

W für Sauerstoff oder Schwefel steht.

Insbesondere bevorzugt sind Verbindungen der Formel (I), in welcher

$R^1$ für gegebenenfalls durch Fluor, Chlor, Brom, Nitro, Methyl, Ethyl, Methoxy, Ethoxy, Halogenalkyl mit 1 bis 4 Kohlenstoffatomen und 1 bis 7 gleichen oder verschiedenen Halogenatomen der Reihe Fluor, Chlor, Brom, Halogenalkoxy mit 1 bis 4 Kohlenstoffatomen und 1 bis 7 gleichen oder verschiedenen Halogenatomen der Reihe Fluor, Chlor, Brom, Alkyl($C_1$-$C_2$)-sulfonyl, Halogenalkyl-($C_1$-$C_2$)sulfonyl mit 1 oder 2 Kohlenstoffatomen und 1 bis 5 gleichen oder verschiedenen Halogenatomen der Reihe Fluor, Chlor, Brom, Halogenalkoxy($C_1$-$C_2$)sulfonyl mit 1 oder 2 Kohlenstoffatomen und 1 bis 5 gleichen oder verschiedenen Halogenatomen der Reihe Fluor, Chlor, Brom substituiertes Phenyl steht oder für gegebenenfalls durch Fluor, Chlor, Methoxy, Ethoxy, Halogenalkoxy($C_1$ -$C_2$) mit 1 oder 2 Kohlenstoffatomen und 1 bis 5 gleichen oder verschiedenen Halogenatomen der Reihe Fluor, Chlor, Brom, substituiertes Alkyl-($C_1$ -$C_3$) steht,

$R^2$ für Wasserstoff, gegebenenfalls durch Fluor, Chlor, Brom, Nitro, Methyl, Ethyl, Methoxy, Ethoxy, Halogenalkyl mit 1 bis 4 Kohlenstoffatomen und 1 bis 5 gleichen oder verschiedenen Halogenatomen der Reihe Fluor, Chlor, Brom, Halogenalkoxy mit 1 bis 4 Kohlenstoffatomen und 1 bis 7 gleichen oder verschiedenen Halogenatomen der Reihe Fluor, Chlor, Brom, Alkyl($C_1$-$C_3$)sulfonyl, Halogenalkyl($C_1$-$C_3$)sulfonyl substituiertes Phenyl steht, für gegebenenfalls durch Fluor, Chlor, Brom, Methoxy, Ethoxy, Halogenalkoxy mit 1 bis 4 Kohlenstoffatomen und 1 bis 7 gleichen oder verschiedenen Halogenatomen der Reihe Fluor, Chlor, Brom substituiertes Alkyl($C_1$-$C_3$ ) oder für Alkoxy($C_1$-$C_3$)carbonyl steht,

$R^3$ für Wasserstoff, Methyl oder Ethyl steht,

$R^4$ für Wasserstoff, gegebenenfalls durch Fluor, Chlor, Methoxy, Ethoxy, Methyl, Ethyl, Halogenalkyl mit 1 oder 2 Kohlenstoffatomen und 1 bis 5 gleichen oder verschiedenen Halogenatomen der Reihe Fluor, Chlor, Brom oder Halogenalkoxy mit 1 oder 2 Kohlenstoffatomen und 1 bis 5 gleichen oder verschiedenen Halogenatomen der Reihe Fluor, Chlor, Brom substituiertes Phenyl oder für gegebenenfalls durch Fluor, Chlor, Brom, Methoxy, Ethoxy substituiertes Alkyl($C_1$-$C_3$) steht,

$R^5$ für Wasserstoff oder gegebenenfalls durch Fluor, Chlor, Methoxy, Ethoxy substituiertes Alkyl($C_1$-$C_3$) steht,

$R^6$ für Trifluormethyl, Chlor-di-fluormethyl, 2,2,2-Trifluorethyl, Pentafluorethyl, Difluormethyl, 1,2,2,2-Tetrafluorethyl, 1,1,2,3,3,3-Hexafluorpropyl, Trifluormethoxy, Chlor-di-fluormethoxy, 2,2,2-Trifluorethoxy, Pentafluorethoxy, Difluormethoxy, 1,2,2,2-Tetrafluorethoxy, 1,1,2,3,3,3-Hexafluorpropoxy steht.

Verwendet man beispielsweise 3,4-Bis-(4-chlorphenyl)-4,5-dihydropyrazol und 4-Trifluormethylcyclohexylisocyanat als Ausgangssubstanzen, so kann der Reaktionsverlauf wie folgt dargestellt werden:

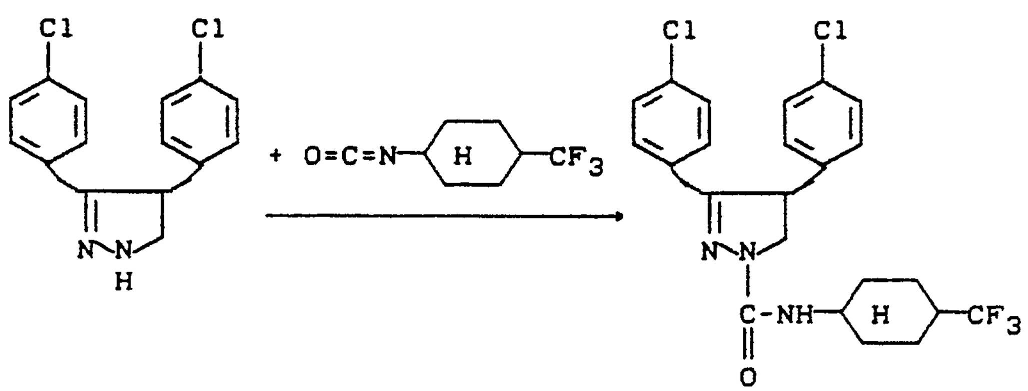

Die zur Durchführung des erfindungsgemäßen Verfahrens als Ausgangsstoffe verwendeten Pyrazolinderivate sind durch die Formel (II) allgemein definiert. In dieser Formel (II) stehen $R^1$, $R^2$, $R^3$, $R^4$ und $R^5$ vorzugsweise für diejenigen Reste, die bereits im Zusammenhang mit der Beschreibung der erfindungsgemäßen Stoffe der Formel (I) als bevorzugt für diese Substituenten genannt werden.

Die Pyrazolinderivate der Formel (II) sind bekannt oder können nach an sich bekannten Methoden hergestellt werden. Siehe dazu J. Agric Food Chem. Vol.25, No.5, (1977) S.987, J. Agric. Food Chem. Vol.27, No.2, (1979), S.406, J. Agric. Food Chem. Vol.26, No.4, (1978), S.915, US.P. 4 439 440; EP-A 113 213; EP-A 058 424; US.P. 4 663 341; DE-A 3 628 647; EP-A 286 346.

Die als Ausgangsverbindungen beim erfindungsgemäßen Verfahren eingesetzten Isocyanate bzw. Thioisocyanate sind durch die Formel (III) allgemein definiert. In dieser Formel (III) stehen W und $R^6$ vorzugsweise für diejenigen Reste, die bereits im Zusammenhang mit der Beschreibung der erfindungsgemäßen Stoffe der Formel (I) als bevorzugt für diese Substituenten genannt werden. Die Verbindungen der Formel (III) sind bekannt oder können nach an sich bekannten Methoden hergestellt werden. Siehe dazu DE-A 2 528 162.

Die Umsetzung von Verbindungen der Formel (II) mit Verbindungen der Formel (III) wird vorzugsweise in einem organischen Lösungsmittel oder Lösungsmittelgemisch bei Temperaturen von 0 bis 150 °C, vorzugsweise bei 20 bis 90 °C umgesetzt. Als organische Lösungsmittel kommen alle für die Umsetzung inerten organischen Solventien in Frage, vorzugsweise werden Ether wie Diethylether, Tetrahydrofuran, Diisopropylether oder Nitrile, wie Acetonitril eingesetzt. Man kann bei Normaldruck oder unter erhöhtem Druck arbeiten, vorzugsweise setzt man unter Normaldruck um. Gegebenenfalls wird die Reaktion in Gegenwart von crganischen oder anorganischen Basen durchgeführt. Als organische Basen kommen vorzugsweise Amine wie Triethylamin, Trimethylamin, Diethylamin, Anilin, Methananilin, Dimethylamin etc. in Frage. Als anorganische Basen können alle üblichen anorganischen Basen wie NaOH, KOH, $Na_2CO_3$, $NaHCO_3$, $K_2CO_3$, Ammoniak etc. eingesetzt werden. Die Reaktionspartner der Verbindungen der Formel (II) und Verbindungen der Formel (III) werden vorzugsweise in äquimolaren Verhältnissen eingesetzt, es kann aber auch ein Überschuß, vorzugsweise ein leichter Überschuß eines Reaktionspartners zugegeben werden.

Die Aufarbeitung der Reaktionsprodukte erfolgt nach an sich üblichen Methoden.

Die Wirkstoffe eignen sich zur Bekämpfung von tierischen Schädlingen, vorzugsweise Arthropoden und Nematoden, insbesondere Insekten und Spinnentieren, die in der Landwirtschaft, in der Tierhaltung, in Forsten, im Vorrats- und Materialschutz sowie auf dem Hygienesektor vorkommen. Sie sind gegen normal sensible und resistente Arten sowie gegen alle oder einzelne Entwicklungsstadien wirksam. Zu den oben erwähnten Schädlingen gehören:

Aus der Ordnung der Isopoda z.B. Oniscus asellus, Armadillidium vulgare, Porcellio scaber.

Aus der Ordnung der Diplopoda z.B. Blaniulus guttulatus.

Aus der Ordnung der Chilopoda z.B. Geophilus carpophagus, Scutigera spec.

Aus der Ordnung der Symphyla z.B. Scutigerella immaculata.

Aus der Ordnung der Thysanura z.B. Lepisma saccharina.

Aus der Ordnung der Collembola z.B. Onychiurus armatus.

Aus der Ordnung der Orthoptera z.B. Blatta orientalis, Periplaneta americana, Leucophaea maderae, Blattella germanica, Acheta domesticus, Gryllotalpa spp., Locusta migratoria migratorioides, Melanoplus differentialis, Schistocerca gregaria.

Aus der Ordnung der Dermaptera z.B. Forficula auricularia.

Aus der Ordnung der Isoptera z.B. Reticulitermes spp..

Aus der Ordnung der Anoplura z.B. Phylloxera vastatrix, Pemphigus spp., Pediculus humanus corporis, Haematopinus spp., Linognathus spp.

Aus der Ordnung der Mallophaga z.B. Trichodectes spp., Damalinea spp.

Aus der Ordnung der Thysanoptera z.B. Hercinothrips femoralis, Thrips tabaci.

Aus der Ordnung der Heteroptera z.B. Eurygaster spp., Dysdercus intermedius, Piesma quadrata, Cimex lectularius, Rhodnius prolixus, Triatoma spp.

Aus der Ordnung der Homoptera z.B. Aleurodes brassicae, Bemisia tabaci, Trialeurodes vaporariorum, Aphis gossypii, Brevicoryne brassicae, Cryptomyzus ribis, Aphis fabae, Doralis pomi, Eriosoma lanigerum, Hyalopterus arundinis, Macrosiphum avenae, Myzus spp., Phorodon humuli, Rhopalosiphum padi, Empoasca spp., Euscelis bilobatus, Nephotettix cincticeps, Lecanium corni, Saissetia oleae, Laodelphax striatellus, Nilaparvata lugens, Aonidiella aurantii, Aspidiotus hederae, Pseudococcus spp. Psylla spp.

Aus der Ordnung der Lepidoptera z.B. Pectinophora gossypiella, Bupalus piniarius, Cheimatobia brumata, Lithocolletis blancardella, Hyponomeuta padella, Plutella maculipennis, Malacosoma neustria, Euproctis chrysorrhoea, Lymantria spp. Bucculatrix thurberiella, Phyllocnistis citrella, Agrotis spp., Euxoa spp., Feltia spp., Earias insulana, Heliothis spp., Spodoptera exigua, Mamestra brassicae, Panolis flammea,

Prodenia litura, Spodoptera spp., Trichoplusia ni, Carpocapsa pomonella, Pieris spp., Chilo spp., Pyrausta nubilalis, Ephestia kuehniella, Galleria mellonella, Tineola bisselliella, Tinea pellionella, Hofmannophila pseudospretella, Cacoecia podana, Capua reticulana, Choristoneura fumiferana, Clysia ambiguella, Homona magnanima, Tortrix viridana.

Aus der Ordnung der Coleoptera z.B. Anobium punctatum, Rhizopertha dominica, Acanthoscelides obtectus, Acanthoscelides obtectus, Hylotrupes bajulus, Agelastica alni, Leptinotarsa decemlineata, Phaedon cochleariae, Diabrotica spp., Psylliodes chrysocephala, Epilachna varive stis, Atomaria spp., Oryzaephilus surinamensis, Antho nomus spp., Sitophilus spp., Otiorrhynchus sulcatus, Cosmopolites sordidus, Ceuthorrhynchus assimilis, Hypera postica, Dermestes spp., Trogoderma spp., Anthrenus spp., Attagenus spp., Lyctus spp., Meligethes aeneus, Ptinus spp., Niptus hololeucus, Gibbium psylloides, Tribolium spp., Tenebrio molitor, Agriotes spp., Cono derus spp., Melolontha melolontha, Amphimallon solsti tialis, Costelytra zealandica.

Aus der Ordnung der Hymenoptera z.B. Diprion spp., Hoplocampa spp., Lasius spp., Monomorium pharaonis, Vespa spp.

Aus der Ordnung der Diptera z.B. Aedes spp., Anopheles spp., Culex spp., Drosophila melanogaster, Musca spp., Hydrotaea spp., Haematobia spp., Glossina spp., Melophagus spp., Fannia spp., Calliphora erythrocephala, Lucilia spp., Chrysomyia spp., Cuterebra spp., Gastrophilus spp., Hyppobosca spp., Stomoxys spp., Oestrus spp., Hypoderma spp., Tabanus spp., Tannia spp., Bibio hortulanus, Oscinella frit, Phorbia spp., Pegomyia hyoscyami, Ceratitis capitata, Dacus oleae, Tipula paludosa.

Aus der Ordnung der Siphonaptera z.B. Xenopsylla cheopis, Ceratophyllus spp., Ctenocephalides spp..

Aus der Ordnung der Arachnida z.B. Scorpio maurus, Latrodectus mactans.

Aus der Ordnung der Acarina z.B. Acarus siro, Argas spp., Ornithodoros spp., Dermanyssus gallinae, Ornithonyssus spp., Eriophyes ribis, Phyllocoptruta oleivora, Boophilus spp., Rhipicephalus spp., Amblyomma spp., Hyalomma spp., Ixodes spp., Dermacentor spp., Haemaphysalis spp., Otobius spp., Psoroptes spp., Chorioptes spp., Sarcoptes spp., Psorergates spp., Demodex spp., Notoedres spp., Tarsonemus spp., Bryobia praetiosa, Panonychus spp., Tetranychus spp..

Die erfindungsgemäßen Wirkstoffe wirken nicht nur gegen Pflanzen-, Hygiene- und Vorratsschädlinge, sondern auch auf dem veterinärmedizinischen Sektor gegen tierische Parasiten (Ektoparasiten und Endoparasiten) wie Schildzecken, Lederzecken, Räudemilben, Laufmilben, Fliegen (stechend und leckend), parasitierende Fliegenlarven, Läuse, Haarlinge, Federlinge, Flöhe und endoparasitisch lebende Würmer.

Sie sind gegen normalsensible und resistente Arten und Stämme sowie gegen alle parasitierenden und nicht parasitierenden Entwicklungsstadien der Ekto- und Endoparasiten wirksam.

Die erfindungsgemäßen Wirkstoffe zeichnen sich durch eine hohe insektizide und akarizide Wirksamkeit aus.

Sie lassen sich mit besonders gutem Erfolg zur Bekämpfung von pflanzenschädigenden Insekten, wie beispielsweise gegen die grüne Pfirsichblattlaus (Myzus persicae) oder gegen die Larven des Meerettichblattkäfers (Phaedon cochleariae) oder gegen die Larven der Kohlschabe (Plutella xylostella) oder gegen die Larven der grünen Reiszikade (Nephotettix cincticeps) oder gegen die Larven des Baumwollkapselwurms (Heliothis armigera) oder gegen die Larven des Heerwurms (Spodoptera frugiperda); zur Bekämpfung von pflanzenschädigenden Milben, wie beispielsweise gegen die gemeine Spinnmilbe oder die Bohnenspinnmilbe (Tetranychus urticae) einsetzen.

Darüberhinaus lassen sie sich mit besonders gutem Erfolg zur Bekämpfung von parasitisch lebenden Warmblüterschädlingen, wie beispielsweise gegen die Larven der Goldfliege (Lucilia cuprina), gegen Rinderzecken (Boophilus microplus) oder gegen Räudemilben (Psoroptes ovis) sowie gegen Schaben (Blattella germanica u.a.) einsetzen.

Weiterhin zeigen die erfindungsgemäßen Verbindungen eine Wirksamkeit gegenüber parasitischen Protozoen und zwar insbesondere gegen Coccidien und/oder Plasmodium.

Die Wirkstoffe können in die übliche Formulierungen übergeführt werden, wie Lösungen, Emulsionen, Suspensionen, Pulver, Schäume, Pasten, Granulate, Aerosole, Wirkstoff-imprägnierte Natur- und synthetische Stoffe, Feinstverkapselungen in polymeren Stoffen und in Hüllmassen für Saatgut, ferner in Formulierungen mit Brennsätzen, wie Räucherpatronen, -dosen, -spiralen u.ä., sowie ULV-Kalt- und Warmnebel-Formulierungen.

Diese Formulierungen werden in bekannter Weise hergestellt, z.B. durch Vermischen der Wirkstoffe mit Streckmitteln, also flüssigen Lösungsmitteln, unter Druck stehenden verflüssigten Gasen und/oder festen Trägerstoffen, gegebenenfalls unter Verwendung von oberflächenaktiven Mitteln, also Emulgiermitteln und/oder Dispergiermitteln und/oder schaumerzeugenden Mitteln. Im Falle der Benutzung von Wasser als Streckmittel können z.B. auch organische Lösungsmittel als Hilfslösungsmittel verwendet werden. Als flüssige Lösungsmittel kommen im wesentlichen in Frage: Aromaten, wie Xylol, Toluol, oder Alkylnaphthaline, chlorierte Aromaten oder chlorierte aliphatische Kohlenwasserstoffe, wie Chlorbenzole, Chlorethylene oder Methylenchlorid, aliphatische Kohlenwasserstoffe, wie Cyclohexan oder Paraffine, z.B. Erdölfraktionen, Alkohole, wie Butanol oder Glycol sowie deren Ether und Ester, Ketone, wie Aceton, Methylethylketon, Methylisobutylketon oder Cyclohexanon, stark polare Lösungsmittel, wie Dimethylformamid und Dimethylsulfoxid, sowie Wasser; mit verflüssigten gasförmigen Streckmitteln oder Trägerstoffen sind solche Flüssigkeiten gemeint, welche bei normaler Temperatur und unter Normaldruck gasförmig sind, z.B. Aerosol-Treibgase, wie Halogenkohlenwasserstoffe sowie Butan, Propan, Stickstoff und Kohlendioxid; als feste Trägerstoffe kommen in Frage: z.B. natürliche Gesteinsmehle, wie Kaoline, Tonerden, Talkum, Kreide, Quarz, Attapulgit, Montmorillonit oder Diatomeenerde und synthetische Gesteinsmehle, wie hochdisperse Kieselsäure, Aluminiumoxid und Silikate; als feste Trägerstoffe für Granulate kommen in Frage: z.B. gebrochene und fraktionierte natürliche Gesteine wie Calcit, Marmor, Bims, Sepiolith, Dolomit sowie synthetische Granulate aus anorganischen und organischen Mehlen sowie Granulate aus organischem Material wie Sägemehl, Kokosnußschalen, Maiskolben und Tabakstengel; als Emulgier und/oder schaumerzeugende Mittel kommen in Frage: z.B. nichtionogene und anionische Emulgatoren, wie Polyoxyethylen-Fettsäure-Ester, Polyoxyethylen-Fettalkohol-Ether, z.B. Alkylarylpolyglykol-Ether, Alkylsulfonate, Alkylsulfate, Arylsulfonate sowie Eiweißhydrolysate; als Dispergiermittel kommen in Frage: z.B. Lignin-Sulfitablaugen und Methylcellulose.

Es können in den Formulierungen Haftmittel wie Carboxymethylcellulose, natürliche und synthetische pulverige, körnige oder latexförmige Polymere verwendet werden, wie Gummiarabicum, Polyvinylalkohol, Polyvinylacetat, sowie natürliche Phospholipide, wie Kephaline und Lecithine, und synthetische Phospholipide. Weitere Additive können mineralische und vegetabile Öle sein.

Es können Farbstoffe wie anorganische Pigmente, z.B. Eisenoxid, Titanoxid, Ferrocyanblau und organische Farbstoffe, wie Alizarin-, Azo- und Metallphthalocyaninfarbstoffe und Spurennährstoffe wie Salze von Eisen, Mangan, Bor, Kupfer, Kobalt, Molybdän und Zink verwendet werden.

Die Formulierungen enthalten im allgemeinen zwischen 0,1 und 95 Gewichtsprozent Wirkstoff, vorzugsweise zwischen 0,5 und 90 %.

Die erfindungsgemäßen Wirkstoffe können in ihren handelsüblichen Formulierungen sowie in den aus diesen Formulierungen bereiteten Anwendungsformen in Mischung mit anderen Wirkstoffen, wie Insektiziden, Lockstoffen, Sterilantien, Akariziden, Nematiziden, Fungiziden, wachstumsregulierenden Stoffen oder Herbiziden vorliegen. Zu den Insektiziden zählen beispielsweise Phosphorsäureester, Carbamate, Carbonsäureester, chlorierte Kohlenwasserstoffe, Phenylharnstoffe, durch Mikroorganismen hergestellte Stoffe u.a..

Die erfindungsgemäßen Wirkstoffe können ferner in ihren handelsüblichen Formulierungen sowie in den aus diesen Formulierungen bereiteten Anwendungsformen in Mischung mit Synergisten vorliegen. Synergisten sind Verbindungen, durch die die Wirkung der Wirkstoffe gesteigert wird, ohne daß der zugesetzte Synergist selbst aktiv wirksam sein muß.

Der Wirkstoffgehalt der aus den handelsüblichen Formulierungen bereiteten Anwendungsformen kann in weiten Bereichen variieren. Die Wirkstoffkonzentration der Anwendungsformen kann von 0,0000001 bis zu 95 Gew.-% Wirkstoff, vorzugsweise zwischen 0,0001 und 1 Gew.-% liegen.

Die Anwendung geschieht in einer den Anwendungsformen angepaßten üblichen Weise.

Bei der Anwendung gegen Hygiene- und Vorratsschädlinge zeichnen sich die Wirkstoffe durch eine hervorragende Residualwirkung auf Holz und Ton sowie durch eine gute Alkalistabilität auf gekälkten Unterlagen aus.

Die erfindungsgemäßen Wirkstoffe eignen sich auch zur Bekämpfung von Insekten, Milben, Zecken usw. auf dem Gebiet der Tierhaltung und Viehzucht, wobei durch die Bekämpfung der Schädlinge bessere Ergebnisse, z.B. höhere Milchleistungen, höheres Gewicht, schöneres Tierfell, längere Lebensdauer usw. erreicht werden können.

Die Anwendung der erfindungsgemäßen Wirkstoffe geschieht auf diesem Gebiet in bekannter Weise, wie durch äußerliche Anwendung in Form beispielsweise des Tauchens (Dippen), Sprühens (Sprayen), Aufgießen (pour-on and spot-on) und des Einpuderns sowie durch parenterale Anwendung in Form beispielsweise der Injektion sowie ferner durch das "feed-through"-Verfahren. Daneben ist auch eine Anwendung als Formkörper (Halsband, Ohrmarke), sowie eine Anwendung in Form der sogenannten Umgebungsbehandlung möglich.

Die biologische Wirksamkeit der erfindungsgemäßen Verbindungen soll anhand der Beispiele A, B und C erläutert werden.

Herstellungsbeispiele

Beispiel 1

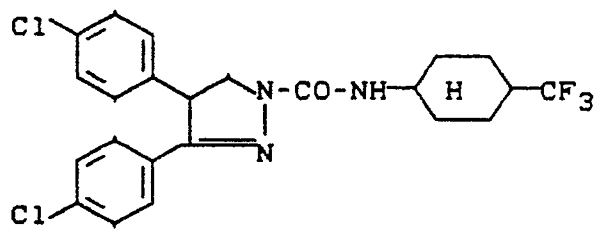

14,5 g (0,05 Mol) 3,4-Bis-(4-chlorphenyl)-4,5-dihydropyrazol werden in 160 ml wasserfreiem Diethylether gelöst, mit 9,7 g cis-trans-4-Trifluormethylcyclohexylisocyanat versetzt und unter Rühren 3 Tropfen Triethylamin zugegeben. Anschließend erhitzt man unter Rühren noch 30 Minuten zum Rückfluß. Man läßt die Reaktionsmischung dann noch 10 Stunden bei Raumtemperatur stehen und saugt dann die ausgefallenen Kristalle ab. Man erhält so 8,1 g eines Isomeren vom 3,4-Bis-(4-chlorphenyl)-4,5-dihydro-1-pyrazolcarbonsäure-(4-trifluormethylcyclohexylamid) als farblose Kristalle mit dem Schmelzpunkt 178° C.

Beispiel 2

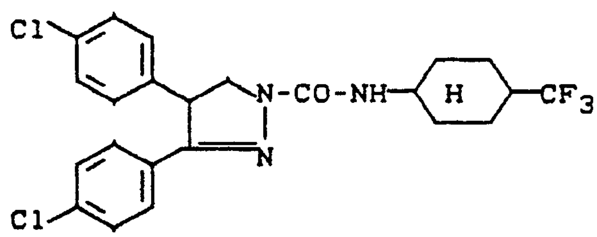

14,5 g (0,05 Mol) 3,4-Bis-(4-chlorphenyl)-4,5-dihydropyrazol werden in 20 ml wasserfreiem Acetonitril gelöst und unter Rühren 9,7 g cis/trans-4-Trifluormethyl-cyclohexylisocyanat zugegeben. Die Mischung wird auf 50° C erhitzt und dann werden 2 Tropfen Triethylamin zugegeben. Anschließend wird bei 50° C eine halbe Stunde lang gerührt. Nach dem Abkühlen wird das Lösungsmittel im Vakuum abdestilliert. Man erhält 24 g 3,4-Bis-(4-chlorphenyl)-4,5-dihydro-1-pyrazolcarbonsäure-cis/trans-(4-trifluormethyl-cyclohexylamid) als gelbes Öl.

$^1$H-NMR ($CDCl_3$, TMS, ppm):
7,52-7,09 (8H,m)
6,22 und 5,93 (1H, 2 x d)
4,6-4,7 (1H,m), 4,3-4,42 (1H,m), 3,95-4,01 (1H,m)
4,05-4,14 und 3,65-3,8 (1H, 2 x d)
1,25-2,32 (9H,m)

In Analogie zu Beispiel 1 und 2 sind die folgenden Verbindungen erhältlich:
Allgemeine Formel:

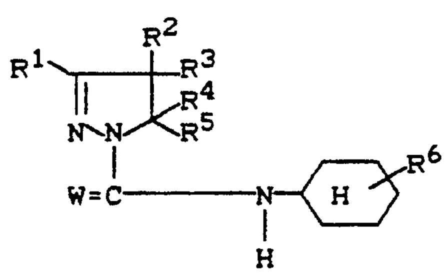

| Beispiel Nr. | $R^1$ | $R^2$ | $R^3$ | W | $R^4$ | $R^5$ | $R^6$ | Fp. |
|---|---|---|---|---|---|---|---|---|
| 3 | 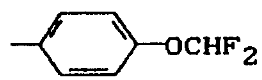 | 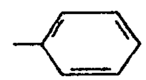 | H | O | H | H | 4-$CF_3$ | 145° C |
| 4 | 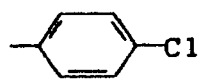 | 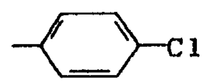 | H | O | H | H | 3-$CF_3$ | 140° C |
| 5 | 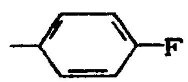 | 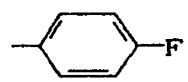 | H | O | H | H | 3-$CF_3$ | Öl |
| 6 | 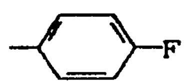 | 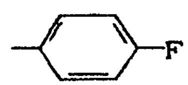 | H | O | H | H | 4-$CF_3$ | Öl |
| 7 | 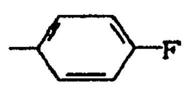 | 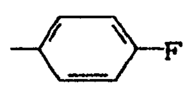 | H | S | H | H | 4-$CF_3$ | Öl |
| 8 | 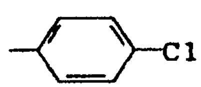 | 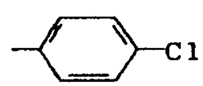 | H | S | H | H | 4-$CF_3$ | Öl |

| Beispiel Nr. | $R^1$ | $R^2$ | $R^3$ | W | $R^4$ | $R^5$ | $R^6$ | Fp. |
|---|---|---|---|---|---|---|---|---|
| 9 | 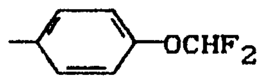 | 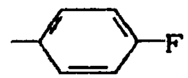 | H | O | H | H | 4-$CF_3$ | Öl |
| 10 | 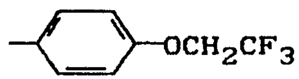 | 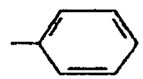 | H | O | H | H | 4-$CF_3$ | |
| 11 | 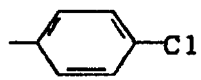 | 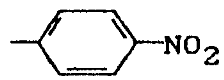 | H | O | H | H | 4-$CF_3$ | |
| 12 | 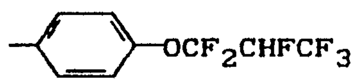 | 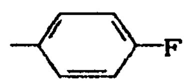 | H | O | H | H | 4-$CF_3$ | |
| 13 | 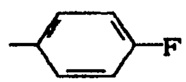 | 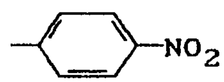 | H | O | H | H | 4-$CF_3$ | |
| 14 | 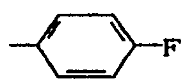 | 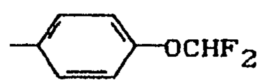 | H | O | H | H | 4-$CF_3$ | |

| Beispiel Nr. | $R^1$ | $R^2$ | $R^3$ | W | $R^4$ | $R^5$ | $R^6$ | Fp. |
|---|---|---|---|---|---|---|---|---|
| 15 | 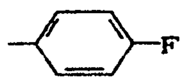 | 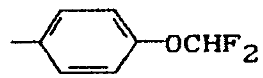 | H | S | H | H | 4-$CF_3$ | |
| 16 | 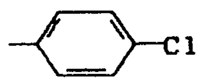 | 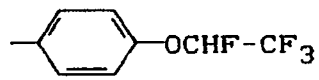 | H | O | H | H | 4-$CF_3$ | |
| 17 | 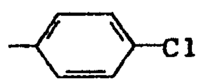 | 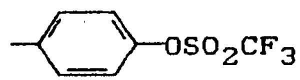 | H | O | H | H | 4-$CF_3$ | |
| 18 | 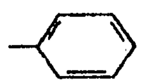 | 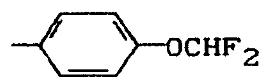 | H | O | H | H | 4-$CF_3$ | |
| 19 | 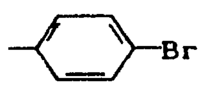 | 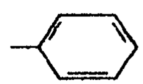 | H | O | H | H | 4-$CF_3$ | |
| 20 | tert.-$C_4H_9$ | H | H | O | H | H | 4-$CF_3$ | Öl |

| Beispiel Nr. | $R^1$ | $R^2$ | $R^3$ | W | $R^4$ | $R^5$ | $R^6$ | Fp. |
|---|---|---|---|---|---|---|---|---|
| 21 | 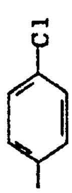 | $COOCH_3$ | $CH_3$ | O | H | H | $4-CF_3$ | |
| 22 | 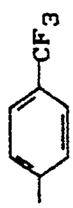 | $COOCH_3$ | $CH_3$ | O | H | H | $4-CF_3$ | |
| 23 | 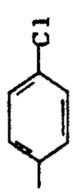 | H | H | O | H | H | $4-CF_3$ | 154° C |
| 24 | 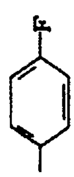 | H | H | O | H | H | $4-CF_3$ | |
| 25 | 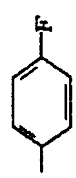 | 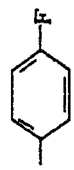 | H | O | H | H | $4-O-CH_2-CF_3$ | Öl |
| 26 | 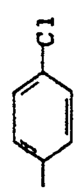 | 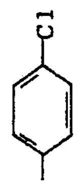 | H | O | H | H | $4-O-CH_2-CF_3$ | |
| 27 | 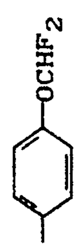 | 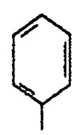 | H | O | H | H | $4-O-CH_2-CF_3$ | |
| 28 | 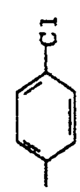 | 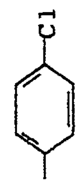 | H | O | $CH_3$ | $CH_3$ | $4-CF_3$ | |
| 29 | 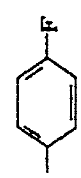 | H | H | O | 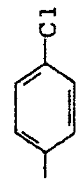 | H | $4-CF_3$ | |

Anwendungsbeispiele

In den folgenden Anwendungsbeispielen wurde die nachfolgend aufgeführte Verbindung als Vergleichssubstanz eingesetzt:

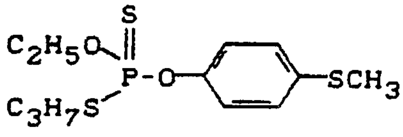

Merpafos = [O-Ethyl-S-propyl-O-(4-methylmercaptophenyl)thionophosphorsäureester]

Beispiel A

Phaedon-Larven-Test

Lösungsmittel: 7 Gewichtsteile Dimethylformamid
Emulgator: 1 Gewichtsteil Alkylarylpolyglykolether

Zur Herstellung einer zweckmäßigen Wirkstoffzubereitung vermischt man 1 Gewichtsteil Wirkstoff mit der angegebenen Menge Lösungsmittel und der angegebenen Menge Emulgator und verdünnt das Konzentrat mit Wasser auf die gewünschte Konzentration.

Kohlblätter (Brassica oleracea) werden durch Tauchen in die Wirkstoffzubereitung der gewünschten Konzentration behandelt und mit Meerrettichblattkäfer-Larven (Phaedon cochleariae) besetzt, solange die Blätter noch feucht sind.

Nach der gewünschten Zeit wird die Abtötung in % bestimmt. Dabei bedeutet 100 %, daß alle Käferlarven abgetötet wurden; 0 % bedeutet, daß keine Käfer-Larven abgetötet wurden.

Bei diesem Test zeigen z.B. die folgenden Verbindungen der Herstellungsbeispiele überlegene Wirksamkeit gegenüber dem Stand der Technik: (1), (6), (7), (8).

Beispiel B

$LD_{100}$-Test
Testtiere: Sitophilus granarius
Lösungsmittel: 35 Gewichtsteile Ethylenglykolmonomethylether
35 Gewichtsteile Nonylphenolpolyglykolether

Zwecks Herstellung einer geeigneten Formulierung vermischt man drei Gewichtsteile Wirkstoff mit sieben Teilen des oben angegebenen Lösungsmittel-Emulgator-Gemisches und verdünnt das so erhaltene Emulsionskonzentrat mit Wasser auf die jeweils gewünschte Konzentration.

2 ml dieser Wirkstoffzubereitung werden auf Filterpapierschalen (Durchmesser 9,5 cm) pipettiert, die sich in Petrischalen entsprechender Größe befinden. Nach Trocknung der Filterscheibe werden ca. 30 Testtiere in die Petrischalen überführt und abgedeckt.

Der Zustand der Testtiere wird 3 Tage nach Ansetzen der Versuche kontrolliert. Es wird diejenige Zeit ermittelt, die für eine 100%ige knock-down Wirkung erforderlich ist. Wird die $LD_{100}$ nach 6 Stunden nicht erreicht, wird der Prozentsatz der knock-down gegangenen Testtiere festgestellt.

In diesem Test zeigten z.B. die Verbindungen der Herstellungsbeispiele (3) und (5) bei einer beispielhaften Konzentration von 1000 ppm a.i. eine $LD_{100}$ von 100 Minuten.

Beispiel C

Test mit Lucilia cuprina resistent-Larven
Emulgator: 35 Gewichtsteile Ethylenglykolmonomethylether
35 Gewichtsteile Nonylphenolpolyglykolether

Zur Herstellung einer zweckmäßigen Wirkstoffzubereitung vermischt man drei Gewichtsteile Wirkstoff mit sieben Gewichtsteilen des oben angegebenen Gemisches und verdünnnt das so erhaltene Konzentrat mit Wasser auf die jeweils gewünschte Konzentration.

Etwa 20 Lucilia cuprina res.-Larven werden in ein Teströhrchen gebracht, welches ca. 1 $cm^3$ Pferdefleisch und 0,5 ml der Wirkstoffzubereitung enthält. Nach 24 Stunden wird der Abtötungsgrad bestimmt.

Bei diesem Test zeigen z.B. die folgenden Verbindungen der Herstellungsbeispiele eine stark ausgeprägte Wirksamkeit: (1), (3), (7), (8).

**Patentansprüche**

1. Substituierte Pyrazolinderivate der allgemeinen Formel

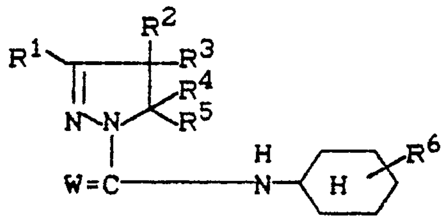

(I)

in welcher

$R^1$ für gegebenenfalls substituiertes Phenyl oder gegebenenfalls substituiertes Alkyl steht,

$R^2$ für Wasserstoff, gegebenenfalls substituiertes Phenyl, gegebenenfalls substituiertes Alkyl oder Alkoxycarbonyl steht,

oder $R^1$ und $R^2$ zusammen für einen gegebenenfalls benzoanellierten Alkylenrest stehen,

$R^3$ für Wasserstoff oder Alkyl steht,

$R^4$ für Wasserstoff, gegebenenfalls substituiertes Phenyl oder gegebenenfalls substituiertes Alkyl steht,

$R^5$ für Wasserstoff oder gegebenenfalls substituiertes Alkyl steht,

$R^6$ für Halogenalkyl oder Halogenalkoxy steht und

W für Sauerstoff oder Schwefel steht.

**2.** Substituierte Pyrazolinderivate der Formel (I) gemäß Anspruch 1, in welcher

$R^1$ für einen gegebenenfalls durch Halogen, Nitro, Alkyl($C_1$-$C_6$), Alkoxy($C_1$-$C_6$), Halogenalkyl-($C_1$-$C_6$), Halogenalkoxy($C_1$-$C_6$), Alkyl($C_1$-$C_6$)-sulfonyl, Halogenalkyl($C_1$-$C_6$)sulfonyl substituiertes Phenyl oder für gegebenenfalls durch Halogen, Alkoxy($C_1$-$C_6$), Halogenalkoxy($C_1$-$C_6$) substituiertes Alkyl($C_1$-$C_6$) steht,

$R^2$ für Wasserstoff, gegebenenfalls durch Halogen, Nitro, Alkyl($C_1$-$C_6$), Alkoxy($C_1$-$C_6$), Halogenalkyl($C_1$-$C_6$), Halogenalkoxy($C_1$-$C_6$), Alkyl-($C_1$-$C_6$)sulfonyl, Halogenalkyl($C_1$-$C_6$)-sulfonyl substituiertes Phenyl, für gegebenenfalls durch Halogen, Alkoxy($C_1$-$C_6$), Halogenalkoxy($C_1$-$C_6$) substituiertes Alkyl($C_1$-$C_6$) oder für Alkoxy($C_1$-$C_6$)carbonyl steht,

$R^3$ für Wasserstoff oder Alkyl($C_1$-$C_6$) steht,

$R^4$ für Wasserstoff, gegebenenfalls durch Halogen, Alkoxy($C_1$-$C_4$), Alkyl($C_1$-$C_4$), Halogenalkyl-($C_1$-$C_4$), Halogenalkoxy($C_1$-$C_4$) substituiertes Phenyl oder für gegebenenfalls durch Halogen, Alkoxy($C_1$-$C_4$) substituiertes Alkyl($C_1$-$C_6$) steht,

$R^5$ für Wasserstoff oder gegebenenfalls durch Halogen, Alkoxy($C_1$-$C_4$) substituiertes Alkyl($C_1$-$C_6$) steht,

$R^6$ für Halogenalkyl($C_1$-$C_6$) oder Halogenalkoxy-($C_1$-$C_6$) steht

und

W für Sauerstoff oder Schwefel steht.

**3.** Substituierte Pyrazolinderivate der Formel (I) gemäß Anspruch 1, in welcher

$R^1$ für gegebenenfalls durch Fluor, Chlor, Brom, Nitro, Alkyl($C_1$-$C_4$), Alkoxy($C_1$-$C_4$), Halogenalkyl-($C_1$-$C_4$), Halogenalkoxy($C_1$-$C_4$), Alkyl-($C_1$-$C_4$)sulfonyl, Halogenalkyl($C_1$-$C_4$)sulfonyl, Halogenalkoxy($C_1$-$C_4$)sulfonyl substituiertes Phenyl oder für gegebenenfalls durch Fluor, Chlor, Brom, Alkoxy($C_1$-$C_4$), Halogenalkoxy($C_1$-$C_4$) substituiertes Alkyl($C_1$-$C_4$) steht,

$R^2$ für Wasserstoff, gegebenenfalls durch Fluor, Chlor, Brom, Nitro, Alkyl($C_1$-$C_4$), Alkoxy-($C_1$-$C_4$), Halogenalkyl($C_1$-$C_4$), Halogenalkoxy($C_1$-$C_4$), Alkyl($C_1$-$C_4$)sulfonyl, Halogenalkyl($C_1$-$C_4$)-sulfonyl substituiertes Phenyl steht, für gegebenenfalls durch Fluor, Chlor, Brom, Alkoxy($C_1$-$C_4$), Halogenalkoxy($C_1$-$C_4$) substituiertes Alkyl($C_1$-$C_4$) oder für Alkoxy($C_1$-$C_4$)carbonyl steht,

$R^3$ für Wasserstoff oder Alkyl($C_1$-$C_4$) steht,

$R^4$ für Wasserstoff, gegebenenfalls durch Fluor, Chlor, Brom, Methoxy, Ethoxy, Methyl, Ethyl, Halogenalkyl($C_1$-$C_2$), Halogenalkoxy($C_1$-$C_2$) substituiertes Phenyl oder für gegebenenfalls durch Fluor, Chlor, Brom, Methoxy, Ethoxy substituiertes Alkyl($C_1$-$C_4$) steht,

$R^5$ für Wasserstoff oder gegebenenfalls durch Fluor, Chlor, Brom, Methoxy, Ethoxy substituiertes Alkyl($C_1$-$C_4$) steht,

$R^6$ für Halogenalkyl mit 1 bis 4 Kohlenstoffatomen und 1 bis 7 gleichen oder verschiedenen Halogenatomen der Reihe Fluor, Chlor, Brom oder für Halogenalkoxy mit 1 bis 4 Kohlenstoffatomen und 1 bis 7 gleichen oder verschiedenen Halogenatomen der Reihe Fluor, Chlor,

Brom steht und

W für Sauerstoff oder Schwefel steht.

4. Substituierte Pyrazolinderivate der Formel (I) gemäß Anspruch 1, in welcher

$R^1$ für gegebenenfalls durch Fluor, Chlor, Brom, Nitro, Methyl, Ethyl, Methoxy, Ethoxy, Halogenalkyl mit 1 bis 4 Kohlenstoffatomen und 1 bis 7 gleichen oder verschiedenen Halogenatomen der Reihe Fluor, Chlor, Brom, Halogenalkoxy mit 1 bis 4 Kohlenstoffatomen und 1 bis 7 gleichen oder verschiedenen Halogenatomen der Reihe Fluor, Chlor, Brom, Alkyl($C_1$-$C_2$)-sulfonyl, Halogenalkyl($C_1$-$C_2$)sulfonyl mit 1 oder 2 Kohlenstoffatomen und 1 bis 5 gleichen oder verschiedenen Halogenatomen der Reihe Fluor, Chlor, Brom, Halogenalkoxy($C_1$-$C_2$)-sulfonyl mit 1 oder 2 Kohlenstoffatomen und 1 bis 5 gleichen oder verschiedenen Halogenatomen der Reihe Fluor, Chlor, Brom substituiertes Phenyl steht oder für gegebenenfalls durch Fluor, Chlor, Methoxy, Ethoxy, Halogenalkoxy($C_1$-$C_2$) mit 1 oder 2 Kohlenstoffatomen und 1 bis 5 gleichen oder verschiedenen Halogenatomen der Reihe Fluor, Chlor, Brom, substituiertes Alkyl($C_1$-$C_3$) steht,

$R^2$ für Wasserstoff, gegebenenfalls durch Fluor, Chlor, Brom, Nitro, Methyl, Ethyl, Methoxy, Ethoxy, Halogenalkyl mit 1 bis 4 Kohlenstoffatomen und 1 bis 5 gleichen oder verschiedenen Halogenatomen der Reihe Fluor, Chlor, Brom, Halogenalkoxy mit 1 bis 4 Kohlenstoffatomen und 1 bis 7 gleichen oder verschiedenen Halogenatomen der Reihe Fluor, Chlor, Brom, Alkyl-($C_1$-$C_3$)sulfonyl, Halogenalkyl($C_1$-$C_3$)sulfonyl substituiertes Phenyl steht, für gegebenenfalls durch Fluor, Chlor, Brom, Methoxy, Ethoxy, Halogenalkoxy mit 1 bis 4 Kohlenstoffatomen und 1 bis 7 gleichen oder verschiedenen Halogenatomen der Reihe Fluor, Chlor, Brom substituiertes Alkyl($C_1$-$C_3$) oder für Alkoxy($C_1$-$C_3$)carbonyl steht,

$R^3$ für Wasserstoff, Methyl oder Ethyl steht,

$R^4$ für Wasserstoff, gegebenenfalls durch Fluor, Chlor, Methoxy, Ethoxy, Methyl, Ethyl, Halogenalkyl mit 1 oder 2 Kohlenstoffatomen und 1 bis 5 gleichen oder verschiedenen Halogenatomen der Reihe Fluor, Chlor, Brom oder Halogenalkoxy mit 1 oder 2 Kohlenstoffatomen und 1 bis 5 gleichen oder verschiedenen Halogenatomen der Reihe Fluor, Chlor, Brom substituiertes Phenyl oder für gegebenenfalls durch Fluor, Chlor, Brom, Methoxy, Ethoxy substituiertes Alkyl($C_1$-$C_3$) steht,

$R^5$ für Wasserstoff oder gegebenenfalls durch Fluor, Chlor, Methoxy, Ethoxy substituiertes Alkyl-($C_1$-$C_3$) steht,

$R^6$ für Trifluormethyl, Chlor-di-fluormethyl, 2,2,2-Trifluorethyl, Pentafluorethyl, Difluormethyl, 1,2,2,2-Tetrafluorethyl, 1,1,2,3,3,3-Hexafluorpropyl, Trifluormethoxy, Chlor-difluormethoxy, 2,2,2-Trifluorethoxy, Pentafluorethoxy, Difluormethoxy, 1,2,2,2-Tetrafluorethoxy, 1,1,2,3,3,3-Hexafluorpropoxy steht.

5. Verfahren zur Herstellung von substituierten Pyrazolinderivaten der allgemeinen Formel (I)

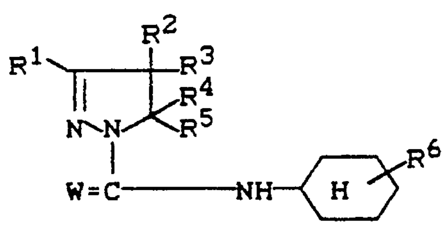

(I)

in welcher

$R^1$ für gegebenenfalls substituiertes Phenyl oder gegebenenfalls substituiertes Alkyl steht,

$R^2$ für Wasserstoff, gegebenenfalls substituiertes Phenyl, gegebenenfalls substituiertes Alkyl oder Alkoxycarbonyl steht,

oder $R^1$ und $R^2$ zusammen für einen gegebenenfalls benzoanellierten Alkylenrest stehen,

$R^3$ für Wasserstoff oder Alkyl steht,

$R^4$ für Wasserstoff, gegebenenfalls substituiertes Phenyl oder gegebenenfalls substituiertes Alkyl steht,

$R^5$ für Wasserstoff oder gegebenenfalls substituiertes Alkyl steht,

$R^6$ für Halogenalkyl oder Halogenalkoxy steht und

W für Sauerstoff oder Schwefel steht,
dadurch gekennzeichnet, daß man Pyrazolinderivate der Formel (II)

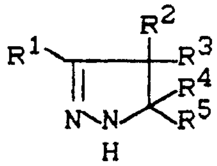

(II)

in welcher
$R^1$, $R^2$, $R^3$, $R^4$ und $R^5$ die oben angegebene Bedeutung besitzen,
mit Isocyanaten bzw. Isothiocyanaten der Formel (III)

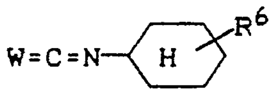

(III)

in welcher
W und $R^6$ die oben angegebene Bedeutung haben,
gegebenenfalls in Gegenwart von Basen umsetzt.

**6.** Schädlingsbekämpfungsmittel, gekennzeichnet durch einen Gehalt an mindestens einem substituierten Pyrazolinderivat der Formel (I).

**7.** Verfahren zur Bekämpfung von tierischen Schädlingen, dadurch gekennzeichnet, daß man substituierte Pyrazolinderivate der Formel (I) auf tierische Schädlinge und/oder ihren Lebensraum einwirken läßt.

**8.** Verwendung von substituierten Pyrazolinderivaten der Formel (I) zur Bekämpfung von tierischen Schädlingen.

**9.** Verfahren zur Herstellung von Schädlingsbekämpfungsmitteln, dadurch gekennzeichnet, daß man substituierte Pyrazolinderivate der Formel (I) mit Streckmitteln und/oder oberflächenaktiven Mitteln vermischt.

Europäisches Patentamt

# EUROPÄISCHER RECHERCHENBERICHT

Nummer der Anmeldung

EP 90124682.7

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int Cl 5) |
|---|---|---|---|
| A | EP - A1 - 0 227 055 (SCHERING AKTIENGESELLSCHAFT) * Ansprüche 1,6-8 * -- | 1,5-9 | C 07 D 231/08<br>C 07 D 231/54<br>A 01 N 43/56 |
| D,A | EP - A2/A3 - 0 113 213 (FBC LIMITED) * Ansprüche 1,11,12 * -- | 1,5-9 | |
| A | DE - A1 - 3 816 062 (SCHERING AG) * Ansprüche 1-5 * -- | 1,5-9 | |
| A | US - A - 4 767 779 (DUGGAN) * Ansprüche 1,29,33 * ---- | 1,5-9 | |

RECHERCHIERTE SACHGEBIETE (Int Cl 5)

C 07 D 231/00
A 01 N 43/00

Der vorliegende Recherchenbericht wurde für alle Patentansprüche erstellt.

| Recherchenort | Abschlußdatum der Recherche | Prüfer |
|---|---|---|
| WIEN | 19-03-1991 | BRUS |

KATEGORIE DER GENANNTEN DOKUMENTEN

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur
T : der Erfindung zugrunde liegende Theorien oder Grundsätze

E : älteres Patentdokument, das jedoch erst am oder nach dem Anmeldedatum veröffentlicht worden ist
D : in der Anmeldung angeführtes Dokument
L : aus andern Gründen angeführtes Dokument

& : Mitglied der gleichen Patentfamilie, übereinstimmendes Dokument

EPA Form 1503 03 82